# EUROPEAN PATENT APPLICATION

(11) **EP 1 547 585 A1**
(43) Date of publication of application: **29.06.2005**
(21) Application number: 03798489.5
(22) Date of filing: 25.09.2003
(51) Int. Cl.: A61K 31/167, A61K 31/4164, A61K 31/4406, A61P 19/02, A61P 29/00

(54) **THERAPEUTIC AGENT FOR RHEUMATISM CONTAINING BENZAMIDE DERIVATIVE AS ACTIVE INGREDIENT**

(30) Priority: 25.09.2002 JP 2002278985
(71) Applicant: SANTEN PHARMACEUTICAL CO., LTD., Higashiyodogawa-ku, Osaka-shi, Osaka 533-8651 (JP)
(72) Inventor: AONO, Hiroyuki, Santen Pharmaceutical Co. Ltd., Osaka-shi, Osaka 533-8651 (JP); OKAMOTO, Masahiro, Santen Pharmaceutical Co. Ltd., Osaka-shi, Osaka 533-8651 (JP); TAKAI, Miwa, Santen Pharmaceutical Co., Ltd., Osaka-shi, Osaka 533-8651 (JP); SETOGUCHI, Chikako, Santen Pharmaceutical Co. Ltd., Osaka-shi, Osaka 533-8651 (JP)
(74) Representative: Peaucelle, Chantal
(86) International application number: PCT/JP2003/012221
(87) International publication number: WO 2004/028526

(57) **Abstract**

An object of the present invention is to provide a therapeutic agent for rheumatic diseases comprising a benzamide derivative as an active ingredient. The benzamide derivative according to the present invention is a compound represented by the following general formula [1] or a salt thereof as an active ingredient. In the formula, R¹, R² and R³, the same or different, are hydrogen, halogen, hydroxyl, lower alkyl, lower alkoxy, aryl, aryl-lower alkyl, aryloxy, amino, amino-lower alkyl, lower alkylamino or carboxyl, X is aryl or a heterocycle, Y is a direct bond or lower alkylene wherein the lower alkylene can contain oxygen atom(s) in the group, and Z is a direct bond or lower alkylene.

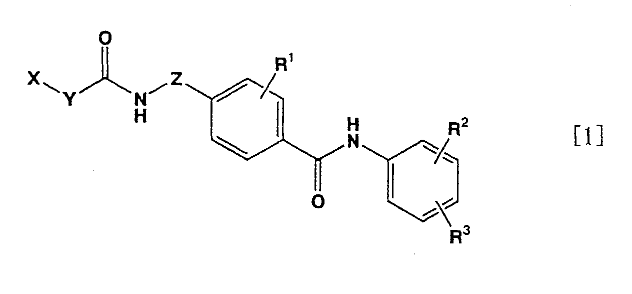

## Description

### Technical Field

The present invention relates to a therapeutic agent for rheumatic diseases comprising a benzamide derivative as an active ingredient.

### Background Art

Rheumatic disease is an intractable chronic inflammatory disease where arthrosynovial membrane is a main site of lesion but there are still many ambiguous points for its onset cause and there are various symptoms as well. One of the characteristics of pathology of the rheumatic disease is abnormal multiplication of synovial membrane and the succeeding destruction of cartilage and bone. Therapy of rheumatic disease is mainly conducted by means of a pharmacotherapy and, with regard to the drugs therefor, steroidal agents and nonsteroidal anti-inflammatory drugs (NSAIDs) have been used for curing the symptoms in rheumatic disease. Further, it has been found recently that systemic immune abnormality such as autoantibody production is observed in patients with rheumatic diseases, and immunosuppressive agent, immunomodulators (DMARDs), etc. have been used.

On the other hand, it is disclosed that benzamide derivatives, which are active ingredients of the present invention, are useful as therapeutic agents for cancer and autoimmune diseases based on a differentiation-inducing action and a histone deacetylase inhibitory action. (See Japanese Laid-open Patent Publication Nos. 152462/1998 and 302173/1999.) These patent publications disclose benzamide derivatives having a large number of combinations of various chemical structures, and the inventions of these patent publications are aimed at treatment of cancer diseases. These patent publications list up many diseases as applicable diseases but have no description of specific pharmacological effects of the compounds, particularly specific effects on rheumatic diseases.

These patent publications do not even suggest what basic chemical structure of benzamide derivatives is useful for rheumatic diseases at all.

Accordingly, it is a very interesting subject to find compounds which are useful as therapeutic agents for rheumatic diseases among these many known benzamide derivatives.

### Disclosure of the Invention

The present inventors precisely studied the effects of benzamide derivatives on treatment of rheumatic diseases.

Various methods of evaluating and studying therapeutic effects on rheumatic diseases using animal models have been reported and are exemplified by a method using collagen-induced arthritis hydroxyl, lower alkyl, lower alkoxy, aryl, aryl-lower alkyl, aryloxy, amino, amino-lower alkyl, lower alkylamino or carboxyl, X is aryl or a heterocycle, Y is a direct bond (i.e., a single bond) or lower alkylene wherein the lower alkylene can contain oxygen atom(s) in the group, and Z is a direct bond (i.e., a single bond) or lower alkylene. The same definitions are applied hereinafter.

The groups defined above are described in more detail. The halogen is fluorine, chlorine, bromine or iodine. The lower alkyl is straight-chain alkyl or branched alkyl, having one to six carbon atoms such as methyl, ethyl, n-propyl, isopropyl, n-butyl, isobutyl, sec-butyl, tert-butyl, n-pentyl, isopentyl, neopentyl, n-hexyl or isohexyl. The lower alkoxy is straight-chain alkoxy,or branched alkoxy having one to six carbon atoms such as methoxy, ethoxy, n-propoxy, isopropoxy, n-butoxy, tert-butoxy, n-pentyloxy or n-hexyloxy. The aryl is a monocyclic aromatic ring or a condensed aromatic ring such as phenyl or naphthyl. The lower alkylene is straight-chain alkylene or branched alkylene having one to six carbon atoms such as methylene, ethylene, methylmethylene, trimethylene, propylene, tetramethylene, ethylethylene, 1,2-dimethylethylene, pentamethylene or hexamethylene. The heterocycle is a five or six-membered monocyclic heterocycle containing one to four nitrogen atom(s), oxygen atom(s) and/or sulfur atom(s) such as pyridine, pyrazine, pyrimidine, pyridazine, thiophene, furan, pyrrol, pyrazol, isooxazol, isothiazole, imidazole, oxazole, thiazole, piperidine, piperazine, pyrrolidine, quinuclidine, tetrahydrofuran, morpholine or thiomorpholine, or a models (Nature, 283, 666-668 (1980)). Accordingly, in the present invention, the effects of benzamide derivatives on rheumatic diseases were evaluated and studied using the above-mentioned animal model. As will be given in the detailed test method and the result thereof which will be mentioned in Examples (under the item of pharmacological test) later, a compound group having a basic chemical structure represented by the following general formula [1] (hereinafter referred to as "the present compound") exhibited remarkable anti-arthritic effects.

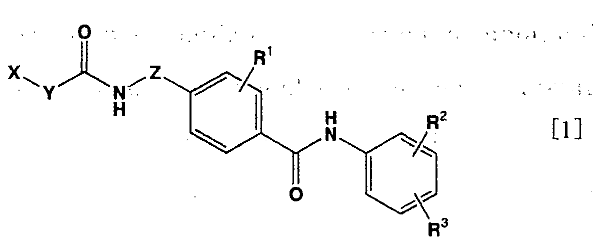

These results show that the present compounds are very useful as therapeutic agents for rheumatic diseases.

The present invention relates to therapeutic agents for rheumatic diseases comprising a compound represented by the following general formula [1] or salts thereof as active ingredients, 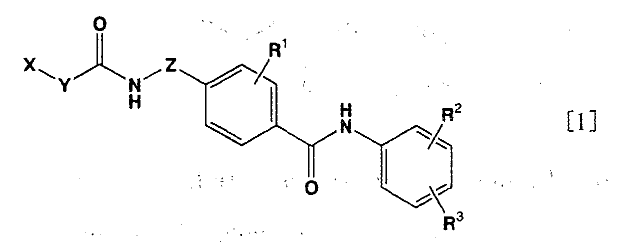 wherein R¹, R² and R³, the same or different, are hydrogen, halogen, condensed heterocycle containing one to four nitrogen atom(s), oxygen atom(s) and/or sulfur atom(s) such as quinoline, isoquinoline, naphthidine, flopyridine, thienopyridine, pyrrolopyridine, oxazolopyridine, imidazolopyridine, thiazolopyridine, benzofuran, benzothiophene or benzimidazole.

The aryl can have substituent(s), and examples of substituents are halogen, hydroxyl, lower alkyl, lower alkoxy, aryl, aryl-lower alkyl, aryloxy, amino, amino-lower alkyl, lower alkylamino, carboxyl or a heterocycle.

The heterocycle can have substituent(s), and examples of substituents are halogen, hydroxyl, lower alkyl, lower alkoxy, aryl, aryl-lower alkyl, aryloxy, amino, amino-lower alkyl, lower alkylamino, carboxyl and a heterocycle.

The lower alkylene containing oxygen atom(s) in the group is straight-chain or branched lower alkylene having one to six carbon atoms and containing oxygen atom(s) such as -OCH₂-, -OCH₂CH₂-, -OCH₂CH₂CH₂- or -CH₂OCH₂-.

The amino or hydroxyl can be protected with a widely-used protecting group. Examples of protecting groups are lower alkanoyl such as acetyl and acyl such as benzoyl.

Further, the carboxyl can be converted into the form of esters. Examples of esters are lower alkyl esters such as a methyl ester and an ethyl ester and aryl-lower alkyl esters such as a benzyl ester.

A preferred example of R¹ and R² in the general formula [1] is hydrogen.

A preferred example of R³ in the general formula [1] is amino.

Preferred examples of X in the general formula [1] are substituted or unsubstituted phenyl and substituted or unsubstituted monocyclic heterocycles having at least heteroatom selected from nitrogen atom(s), oxygen atom(s) and sulfur atom(s), and particularly preferred examples thereof are phenyl, and phenyl and pyridyl substituted by imidazolyl.

Preferred examples of Y in the general formula [1] are a direct bond, -CH₂O- and -CH₂OCH₂-.

Preferred examples of Z in the general formula [1] are a direct bond and methylene.

A preferred example of combinations of the substituents in the general formula [1] is a combination wherein R¹ and R² are hydrogen, R³ is amino, X is phenyl, or phenyl or pyridyl substituted by imidazolyl, Y is a direct bond, -CH₂O- or -CH₂OCH₂-, and Z is a direct bond or methylene.

Particularly preferred specific examples of the present compounds are N-(2-aminophenyl)-4-(N-benzoylaminomethyl)-benzamide [2], N-(2-aminophenyl)-4-{N-[4-(imidazol-1-yl)benzyl]-oxycarbonylaminomethyl}benzamide [3], N-(2-aminophenyl)-4-[N-(pyridin-3-yl)methoxycarbonylaminomethyl]benzamide [4], N-(2-aminophenyl)-4-[N-(pyridin-3-yl)oxyacetylaminomethyl]benzamide [5] and N-(2-aminophenyl)-4-[(pyridin-3-yl)methoxyacetylamino]-benzamide [6] represented by the following chemical formulae and salts thereof.

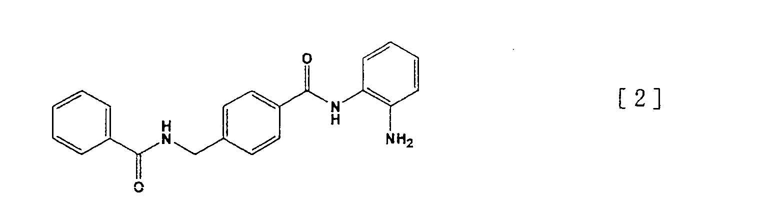

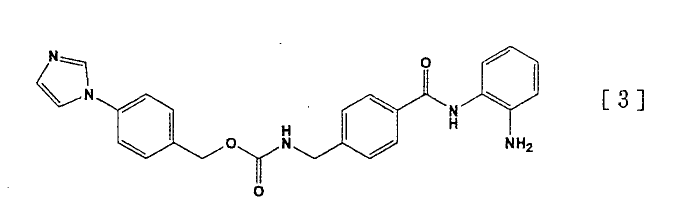

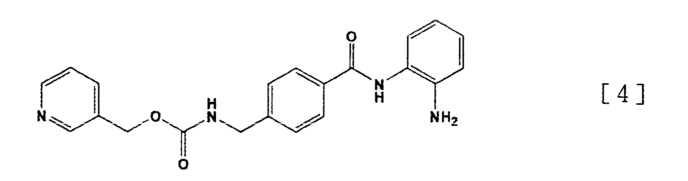

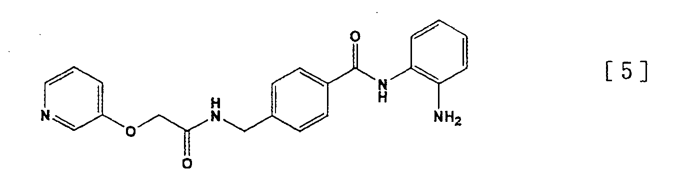

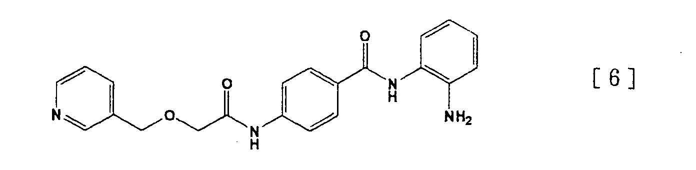

The above-mentioned salts can be any pharmaceutically acceptable salts and are exemplified by salts with an inorganic acid such as hydrochloric acid, nitric acid, sulfuric acid, hydrobromic acid or phosphoric acid, salts with an organic acid such as acetic acid, lactic acid, malic acid, succinic acid, citric acid, benzoic acid, fumaric acid, maleic acid, tartaric acid, trifluoroacetic acid, methanesulfonic acid or p-toluenesulfonic acid, salts with an alkali metal or an alkaline earth metal such as sodium, potassium or calcium and salts with an organic amine such as triethylamine or pyridine.

There are optical isomers in some of the present compounds, and they are also included in the present invention. Further, the present compounds can be in the form of solvates such as hydrates.

There is no need of using special techniques for preparing formulations containing the present compound, and widely-used techniques may be applied for preparing them. Examples of dosage forms are systemic administration preparations such as injections, tablets, capsules, powders and granules and formulations for topical administration such as paints and topical injections.

Solid preparations such as tablets, capsules, powders and granules can be prepared by optionally using a diluent such as lactose, crystalline cellulose or starch, a lubricant such as magnesium stearate or talc, a binder such as hydroxypropylcellulose or polyvinylpyrrolidone, a disintegrator such as calcium carboxymethylcellulose or low-substituted hydroxypropylmethylcellulose or a coating agent such as hydroxypropylmethylcellulose, macrogol or a silicone resin.

Liquid formulations such as injections and topical injections can be prepared by optionally using a solvent such as purified water for injection, physiological saline, a Ringer's solution or vegetable oil, a solubilizing agent such as ethanol, propylene glycol or glycerin, a stabilizer such as sodium pyrosulfite, sodium hydrogensulfite, sodium ascorbate or ethylenediaminetetraacetic acid, a suspending agent such as sodium carboxymethylcellulose, aluminum monostearate or Polysorbate 80, an emulsifier such as polyoxyethylene hydrogenated castor oil or lecithin, a buffer such as sodium phosphate or sodium acetate, an tonicity agent such as sodium chloride or glucose, a soothing agent such as procaine hydrochloride, benzyl alcohol or chlorobutanol or a preservative such as p-hydroxybenzoate.

These formulations can be ones described in Japanese Laid-open Patent Publication Nos. 152462/1998 and 302173/1999 and further can be one in later Examples (under the item of Formulation). Of course, this Formulation does not limit the scope of the present invention.

The present invention also relates to a method of treating rheumatic diseases comprising administering to a patient a pharmaceutically effective amount of the compound represented by the general formula [1] or a salt thereof.

The usually daily dosage is 0.001 to 1,000 mg which can be given in a single dose or several divided doses.

The dosage and administration times can be appropriately adjusted depending on symptoms and age of patients, administration routes and the like.

### Brief Explanation of the Drawing

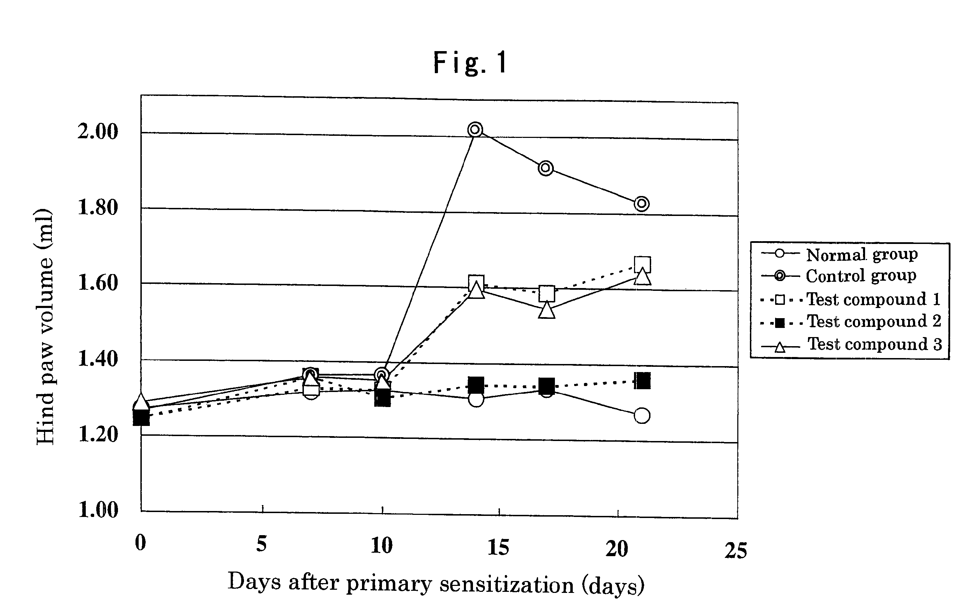

Fig. 1 is a graph showing daily changes of hind paw volume in type II collagen-induced arthritis of rats. □, ■ and Δ show daily changes in 6 mg/kg administrated groups of Test compounds 1, 2 and 3 respectively. ○ and ⓞ show daily changes in an normal group and a control group respectively.

### Best Mode for Carrying out the Invention

Pharmacological tests and Formulation are shown below as Examples. These Examples do not limit the scope of the present invention but are intended to make the present invention more clearly understandable.

### Examples

### Pharmacological Tests

### 1) Effects on type II collagen-induced arthritis

Model of type II collagen-induced arthritis has been widely used as an animal model for evaluating effects of drugs on rheumatic diseases (Nature, 283, 666-668 (1980)). Anti-arthritic effects of the present compounds were evaluated and studied according to this model.

### Experimental method

A solution of bovine joint cartilage-derived type II collagen in 0.05 N acetic acid (2 mg/ml) was mixed with Freund's incomplete adjuvant of the same volume to prepare an emulsion (final concentration: 1 mg/ml).

As a primary sensitization, this emulsion was injected intra-dermally into five sites on the back of each rat (female, seven weeks old, weight: approximately 130 to 160 g) at a dose of 100 µl (total 500 µl: corresponding to 500 µg of type II collagen per rat). Seven days after the primary sensitization, 100 µl of this emulsion was injected intra-dermally into a root of the rat tail again (secondary sensitization).

0.5 ml/kg of a test compound solution (the test compound was dissolved in a 5% carboxymethylcellulose solution as a vehicle) was orally administered immediately before the primary sensitization, 7 days after the primary sensitization and 14 days after the primary sensitization. The dosage of test compound was adjusted to 6 mg/kg.

As the test compound, N-(2-aminophenyl)-4-[N-(pyridin-3-yl)methoxycarbonylaminomethyl]benzamide [chemical formula 4, Test compound 1], N-(2-aminophenyl)-4-[N-(pyridin-3-yl)oxyacetylaminomethyl]benzamide [chemical formula 5, Test compound 2] and N-(2-aminophenyl)-4-[(pyridin-3-yl)methoxyacetylamino]benzamide [chemical formula 6, Test compound 3] were used.

As a control, the vehicle without any test compound was administered by the same manner as above.

### Results

The results of the test are shown in Fig. 1 as the average of eight samples per group. As apparent from Fig. 1, in the control group, hind paw volume began to increase from 3 days after the secondary sensitization (10 days after the primary sensitization) and reached a maximum 7 days after secondary sensitization (14 days after the primary sensitization).

On the other hand, in all the groups administered with the test compound, increases in hind paw volume were remarkably suppressed. Particularly in the case of the rats administrated with test compound 2, any increase in hind paw volume could not be found and the volume was almost the same as that of normal rats.

An example for formulation
Capsule (Amounts of ingredients per capsule)

| | |
|---|---|
| The present compound | 1 mg |
| Lactose | 149 mg |

Desired capsule preparations can be prepared by appropriately changing the combination ratio of the present compound to other additives.

### Industrial Applicability

The present invention provides a therapeutic agent for rheumatic diseases comprising a benzamide derivative as an active ingredient.

## Claims

1. A therapeutic agent for rheumatic diseases comprising a compound represented by the following general formula [1] or a salt thereof as an active ingredient, 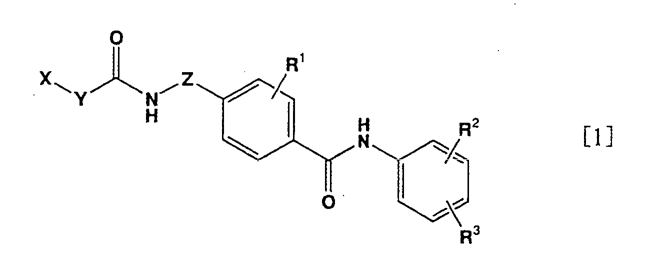 wherein R¹, R² and R³, the same or different, are hydrogen, halogen, hydroxyl, lower alkyl, lower alkoxy, aryl, aryl-lower alkyl, aryloxy, amino, amino-lower alkyl, lower alkylamino or carboxyl, X is aryl or a heterocycle, Y is a direct bond or lower alkylene wherein the lower alkylene can contain oxygen atom(s) in the group, and Z is a direct bond or lower alkylene.

2. The therapeutic agent for rheumatic diseases as claimed in claim 1, wherein R¹ and R² are hydrogen.

3. The therapeutic agent for rheumatic diseases as claimed in claim 1, wherein R³ is amino.

4. The therapeutic agent for rheumatic diseases as claimed in claim 1, wherein the aryl represented by X is substituted or unsubstituted phenyl, and the heterocycle represented by X is a substituted or unsubstituted monocyclic heterocycle having at least one heteroatom selected from the group consisting of nitrogen atom(s), oxygen atom(s) and sulfur atom(s).

5. The therapeutic agent for rheumatic diseases as claimed in claim 1, wherein the aryl is phenyl or phenyl substituted by imidazolyl, and the heterocycle is pyridyl.

6. The therapeutic agent for rheumatic diseases as claimed in claim 1, wherein Y is a direct bond, -CH₂O- or -CH₂OCH₂-.

7. The therapeutic agent for rheumatic diseases as claimed in claim 1, wherein Z is a direct bond or methylene.

8. The therapeutic agent for rheumatic diseases as claimed in claim 1, wherein R¹ and R² are hydrogen, R³ is amino, X is phenyl, or phenyl or pyridyl substituted by imidazolyl, Y is a direct bond, -CH₂O- or -CH₂OCH₂-, and Z is a direct bond or methylene.

9. The therapeutic agent for rheumatic diseases as claimed in claim 1, which comprises N-(2-aminophenyl)-4-(N-benzoylaminomethyl)benzamide, N-(2-aminophenyl)-4-{N-[4-(imidazol-1-yl)benzyl]oxycarbonylaminomethyl}benzamide, N-(2-aminophenyl)-4-[N-(pyridin-3-yl)methoxycarbonylaminomethyl]benzamide, N-(2-aminophenyl)-4-[N-(pyridin-3-yl)oxyacetylaminomethylbenzamide, N-(2-aminophenyl)-4-[(pyridin-3-yl)methoxyacetylamino]benzamide or a salt thereof as an active ingredient.

10. A method of treating rheumatic diseases comprising administering to a patient a pharmaceutically effective amount of a compound represented by the following general formula [1] or a salt thereof, 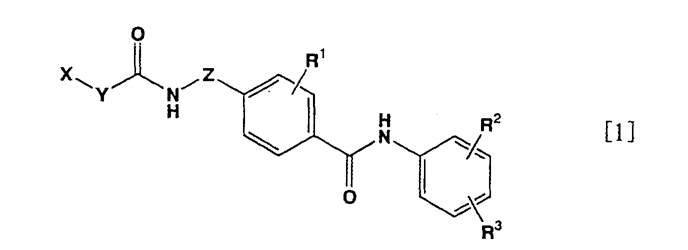 wherein R¹, R² and R³, the same or different, are hydrogen, halogen, hydroxyl, lower alkyl, lower alkoxy, aryl, aryl-lower alkyl, aryloxy, amino, amino-lower alkyl, lower alkylamino or carboxyl, X is aryl or a heterocycle, Y is a direct bond or lower alkylene wherein the lower alkylene can contain oxygen atom(s) in the group, and Z is a direct bond or lower alkylene.

11. Use of a compound represented by the following general formula [1] or a salt thereof in the manufacture of a therapeutic agent for rheumatic diseases, 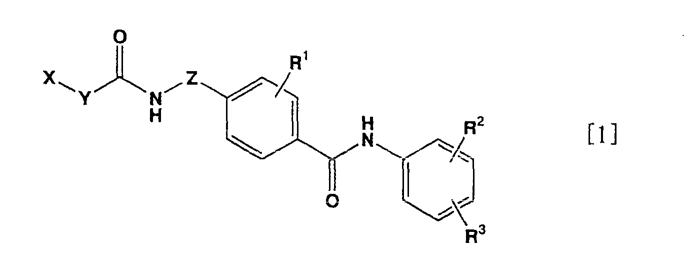 wherein R¹, R² and R³, the same or different, are hydrogen, halogen, hydroxyl, lower alkyl, lower alkoxy, aryl, aryl-lower alkyl, aryloxy, amino, amino-lower alkyl, lower alkylamino or carboxyl, X is aryl or a heterocycle, Y is a direct bond or lower alkylene wherein the lower alkylene can contain oxygen atom(s) in the group, and Z is a direct bond or lower alkylene.
